# FASCICULE DE BREVET EUROPEEN

(11) **EP 1 854 353 B1**
(45) Date de publication et mention de la délivrance du brevet: **03.06.2009**
(21) Numéro de dépôt: 07290480.8
(22) Date de dépôt: 18.04.2007
(51) Int. Cl.: A01N 31/16, A01N 65/00

(54) **Procédé de traitement de réservoirs de stockage contaminés par des mycotoxines**
Verfahren zur Behandlung von Lagertanks, die mit Mycotoxinen kontaminiert sind
Method of treating storage tanks contaminated by mycotoxins

(30) Priorité: 27.04.2006 FR 0603794
(43) Date de publication de la demande: 14.11.2007
(73) Titulaire: XEDA INTERNATIONAL, 13670 Saint Andiol (FR)
(72) Inventeur: Bompeix, Gilbert, 75116 Paris (FR); Sardo, Alberto, 13160 Chateaurenard (FR)
(74) Mandataire: Colombet, Alain André

(56) Documents cités:
- FR-A1- 2 786 664
- TRIPATHI N N ET AL: "TOXICITY OF SOME TERPENOIDS AGAINST FUNGI INFESTING FRUITS AND SEEDS OF CAPSICUM-ANNUUM DURING STORAGE" PHYTOPATOLOGISCHE ZEITSCHRIFT, VERLAG PAUL PAREY, BERLIN, DE, vol. 110, 1984, pages 328-335, XP002233030 ISSN: 0031-9481
- JUGLAL S; GOVINDEN R; ODHAV B: "Spice oils for the control of co-occurring mycotoxin-producing fungi" JOURNAL OF FOOD PROTECTION, vol. 65, no. 4, 2002, pages 683-687, XP009077641 US
- MONTES-BELMONT R ET AL: "Control of Aspergillus flavus in maize with plant essential oils and their components" JOURNAL OF FOOD PROTECTION, vol. 61, no. 5, mai 1998 (1998-05), pages 616-619, XP000886016 ISSN: 0362-028X
- MANSOUR, N ET AL: "Inhibition of surface growth of toxigenic and nontoxigenic aspergilli and penicillia by eugenol, isoeugenol and monolaurin" JOURNAL OF FOOD SAFETY, vol. 16, no. 3, 1996, pages 219-229, XP009077671

## Description

La présente invention concerne l'utilisation de l'eugénol ou de l'huile de girofle pour le traitement des silos de fruits secs, légumes secs, céréales ou oléagineux contaminés par des mycotoxines et/ou les champignons ou moisissures les produisant dans lequel le traitement est appliqué au silo vide par thermonébubisation.

Au niveau mondial, environ 25% des denrées alimentaires sont contaminées par des mycotoxines. Les mycotoxines sont des molécules hautement toxiques, parfois cancérogènes, produites par certains champignons qui contaminent les aliments.

On compte environ une dizaine de mycotoxines contaminant fréquemment les denrées alimentaires, qui sont dangereuses pour l'homme et/ou les animaux.

Les mieux connues aujourd'hui sont les aflatoxines, les ochratoxines, les toxines de *Fusarium,* telles que zéaralénone, trichothécènes et fumonisines, ou encore la patuline.

Les matières premières sont contaminées au niveau du champ et au niveau du stockage dans certaines conditions de température, humidité, conditions de culture, souches présentes, etc.

Les champignons responsables sont certains champignons ou moisissures parasites, tels que *Fusarium, Penicillium* et *Aspergillus.* Les fruits secs, légumes secs, céréales ou oléagineux sont les denrées les plus fréquemment contaminées.

Très résistantes, les mycotoxines se retrouvent dans les produits finis ; elles ne sont détruites ni par le froid ni par la chaleur ; elles sont stables lors des différents procédés de transformation.

Des cas d'intoxication aiguë chez l'homme sont connus depuis le Moyen Age. Les intoxications humaines aiguës dans les pays industrialisés sont maintenant rares, mais il existe un risque non négligeable de développer des lésions, notamment au niveau du foie et des reins, ou des cancers liés à l'ingestion régulière de faibles doses.

Lorsque ces mycotoxines sont consommées par les animaux d'élevage, on peut les retrouver dans la viande (notamment dans les muscles et les abats) et dans le lait, soit en l'état, soit transformées en des composés parfois plus toxiques que la mycotoxine de départ.

Des cas d'intoxication sont répertoriés dans les élevages, tels que des problèmes de fertilité dus à la zéaralénone, le refus de s'alimenter dû au déoxynivalénol, ou encore une toxicité rénale aiguë due à l'ochratoxine A. Le développement des mycotoxines, impossible à éliminer de la nourriture une fois produite, peut donc entraîner des problèmes tout au long de la chaîne alimentaire, avec des répercussions économiques lourdes.

Il est donc essentiel de mettre à disposition des compositions permettant d'empêcher la contamination par ces mycotoxines.

Par ailleurs, pour la décontamination particulière de denrées alimentaires, il est important que ces compositions soient non toxiques et acceptables sur le plan alimentaire.

L'eugénol est un terpène issu de l'huile de girofle. L'utilisation d'une composition contenant de l'eugénol pour le traitement de fruits et légumes après récolte en vue de prolonger leur durée de conservation a été décrite dans la demande FR 98 08 995. Le procédé décrit comprend l'application de ladite composition sur les fruits et légumes.

Par ailleurs, un procédé anti-germinatif de pommes de terre utilisant l'eugénol a été décrit dans la demande FR 98 15 305. Le procédé comprend l'application par immersion ou douchage de la composition ou l'application par thermonébulisation aux pommes de terre.

Néanmoins, l'utilisation potentielle de l'eugénol pour le traitement des réservoirs de stockage de fruits secs, légumes secs, céréales, oléagineux contaminés par les mycotoxines et/ou les champignons ou moisissures les produisant n'a été à ce jour ni décrite ni suggérée.

Les présents inventeurs ont ainsi démontré que les silos de céréales, étaient fortement pollués par des champignons ou des moisissures tels que *Penicillium, Aspergillus, Fusarium,* producteurs de mycotoxines. Cette contamination persiste même lorsque les silos sont vides, de sorte que les nouvelles récoltes sont à nouveau contaminées.

Il est donc important de pouvoir décontaminer ces silos. Néanmoins, étant donnée la taille de ces réservoirs (jusqu'à plusieurs centaines de tonnes), un agent actif extrêmement puissant est nécessaire. La décontamination des silos est également particulièrement difficile en ce que les parois sont poreuses et les mycotoxines et/ou moisissures ou champignons les produisant peuvent s'y loger et ainsi résister à une simple décontamination de l'air ambiant. Il est donc nécessaire de mettre à disposition un procédé extrêmement efficace permettant l'application de l'agent actif même dans les porosités des parois.

Or, aucune méthode de décontamination aisée, peu coûteuse et efficace n'a été mise au point à ce jour.

Les présents inventeurs ont maintenant découvert, et c'est un des objets de la présente invention, que l'eugénol ou l'huile de girofle présentait, de façon tout à fait inattendue, une activité permettant le traitement des silos de stockage de fruits secs, légumes secs, céréales, oléagineux contaminés par des mycotoxines et/ou les champignons ou moisissures les produisant.

Par ailleurs, l'eugénol étant un produit d'origine naturelle, il convient tout particulièrement à la décontamination de denrées alimentaires.

La présente invention concerne donc un procédé pour décontaminer ou empêcher la contamination, par des mycotoxines et/ou champignons ou moisissures les produisant, de silos de fruits secs, légumes secs, céréales ou oléagineux, comprenant l'application auxdits réservoirs dans lesquels les fruits secs, légumes secs, céréales ou oléagineux sont conservés ou destinés à être conservés, d'une composition comprenant l'eugénol, l'isoeugénol, un de leurs sels acceptables sur le plan alimentaire et/ou de l'huile de girofle, ou leurs mélanges, par thermonébulisation, la dite composition étant appliquée au silo vide.

De préférence, lesdites mycotoxines sont choisies parmi l'ochratoxine A, le désoxynivalénol, les aflatoxines (B1, B2, G1, G2, M1, M2), la zéaralénone, les trichothécénes (DAS, toxine D2, nivalénol, fusarénone X), les fumosinines (B1 et B2), la citrinine, l'acide pénicillique, la vomitoxine, la patuline ; plus particulièrement l'ochratoxine A.

Selon un autre aspect de la présente invention, lesdites mycotoxines sont produites par des champignons du genre *Fusarium, Penicillium* et *Aspergillus.*

La production par ces genres de moisissures peut être dépendante des espèces et/ou s'exprimer dans certaines conditions.

Plus particulièrement, les *Penicillia* et *Aspergilli* se développent généralement dans les réservoirs de stockage des fruits secs, légumes secs, céréales, oléagineux. Parmi les *Penicillia,* on peut citer *Penicillium verrucosum* qui peut produire l'ochratoxine A et/ou la citrinine, *Penicillium orantiogriseum* produisant l'acide pénicillique et/ou l'ochratoxine A. *Penicillium citrinum* et *expansum* produisant la citrinine et/ou la patuline. Parmi les *Aspergilli,* on peut citer *l'Aspergillus ochraceus, Aspergillus carbonarius* et *Aspergillus niger* produisant l'ochratoxine A.

Plus particulièrement, les *Fusarium* contaminent les plants, plus principalement les plants de blé.

Parmi les céréales permettant de mettre en oeuvre le procédé selon l'invention, on peut citer le blé, le maïs, le riz, l'orge.

Parmi les oléagineux, on peut citer le tournesol, le colza, l'arachide.

Les céréales ou oléagineux peuvent se présenter sous forme de grains, graines, semences ou plants.

Parmi les fruits secs et légumes secs, on peut citer raisins, café, cacao, haricots, lentilles.

On préfère notamment les céréales ou oléagineux.

La composition selon l'invention peut être appliquée par aspersion ou thermonébulisation.

Selon un aspect particulièrement avantageux, le procédé selon l'invention est mis en oeuvre pour décontaminer ou empêcher la contamination des silos avant remplissage par les fruits secs, légumes secs, céréales ou oléagineux.

Selon un aspect particulièrement préféré, la composition est appliquée à un silo vide.

Selon l'invention, on entend par composition comprenant l'eugénol, isoeugénol ou huile de girofle, ces ingrédients purs ou toute composition diluée comprenant ces ingrédients. Ainsi, la composition pour le procédé selon l'invention comprend, en pourcentage en poids :
- de 15% à 100 % dudit principe actif;
- de 0% à 10 % d'un ou plusieurs agents réduisant l'évaporation du principe actif;
- de 0% à 85 % d'un tensioactif choisi parmi les tensioactifs anioniques, les tensioactifs non ioniques et leurs mélanges ; et
- de 0% à 80 % d'un solvant choisi parmi l'eau, les (C₁-C₆)alcanols, les (C₂-C₆)alkylèneglycol, les poly(C₁-C₆)alkylèneglycol, les esters (C₁-C₆) alkyliques d'acides (C₁-C₆)alcanoïque et leurs mélanges.

Plus préférentiellement, on utilise l'eugénol pur ou l'huile de girofle pure, qui comprend généralement environ 85% d'eugénol.
De façon alternative, on peut également utiliser la composition suivante :
- de 15% à 60% de principe actif ;
- de 1% à 8% d'un ou plusieurs agents réduisant l'évaporation ;
- de 25% à 60% d'un tensioactif ; et
- de 0% à 30% dudit solvant.
   Plus précisément, on peut utiliser la formulation A suivante :

- eugénol : 30% ;
- dipropylèneglycol (DPG) : 50% ;
- tensio-actifs : 5%
- eau : 15 %.

L'un des avantages associé à ces compositions est leur forte teneur en principe actif.

Dans ce qui précède et ce qui suit, les pourcentages sont en poids/volume par rapport au volume total de la composition.

La formulation de la composition traitante dépend de son mode d'application.

Les agents réduisant l'évaporation du principe actif sont connus dans la technique et peuvent être notamment choisis parmi les polyterpènes dispersables dans l'eau, les esters de glycérol de la résine de pin, les gommes laques, les lécithines, les huiles sicatives, l'alcool polyvinylique, la polyvinyle pyrrolidone, les polyacrylates de métaux alcalins, la gomme arabique.

Les tensioactifs ou émulsifiants variés connus en soi.

Selon la présente invention, on entend par "émulsifiant" tout type d'agent habituellement utilisé à cet effet, tels que les alcools gras éthoxylés, les acides gras éthoxylés, les alkylphénols éthoxylés ou tout autre produit non ionique.

Les tensioactifs préférablement utilisés dans le cadre de l'invention sont des tensioactifs anioniques ou non ioniques.

Des exemples de tensioactifs non ioniques utilisables selon l'invention sont notamment :
- le produit de condensation d'un alcool gras aliphatique, de préférence en C₈-C₂₂, avec un oxyde d'alkylène en C₂-C₃. L'oxyde d'alkylène en C₂-C₃ peut être l'oxyde d'éthylène, l'oxyde de propylène, ou bien un mélange d'oxyde d'éthylène et d'oxyde de propylène dans des proportions quelconques. Un exemple de tels tensioactifs est le produit de condensation de l'alcool laurylique (ou alcool n-dodécyclique) avec 30 moles d'oxyde d'éthylène;
- le produit de condensation d'un alkylphénol dans lequel la chaîne alkyle est en C₈-C₂₂ avec un oxyde d'alkylène en C₂-C₃. Là encore, les produits de condensation avec l'oxyde d'éthylène, l'oxyde de propylène ou bien un mélange d'oxyde d'éthylène et l'oxyde de propylène dans des proportions quelconques sont également avantageux. A titre d'exemple de tels tensioactifs, on peut citer le produit de condensation du n-nonylphénol avec 10 moles d'oxyde d'éthylène;
- le produit de condensation d'un acide gras de préférence en C₈-C₂₂ avec un oxyde d'alkylène en C₂-C₃, par exemple l'oxyde d'éthylène ou l'oxyde de propylène ou un mélange d'oxyde d'éthylène et d'oxyde de propylène dans des proportions quelconques. Ces produits de condensation présentent une chaîne alkoxylée au niveau de la fonction hydroxyle du groupe carboxylique. Des tensioactifs préférés de ce groupe sont les produits de condensation obtenus à partir de l'acide ricinoléique avec 10 moles d'oxyde d'éthylène.

Des exemples de tensioactifs anioniques utilisables selon l'invention sont notamment :
- les sels hydrosolubles d'alkylsulfates à longue chaîne, et notamment les sels hydrosolubles de (C₈-C₂₄)alkylsufates, tels que les laurylsulfates de métaux alcalins et plus particulièrement le laurylsulfate de sodium; et
- les sels hydrosolubles d'alkylarylsulfonates et notamment les sels hydrosolubles de (C₈-C₂₄)alkyl-(C₆-C₁₀)arylsulfonates, tels que les dodécyl-benzènesulfonates de métaux alcalins et plus particulièrement le dodécyl-benzènesulfonate de sodium.

L'invention n'est cependant pas limitée à l'utilisation de ces tensioactifs particuliers.

Les solvants pouvant être utilisés dans la composition traitante sont notamment choisis parmi les alcools aliphatiques en C₁-C₁₂, les glycols et les esters alkyliques d'acides carboxyliques.

Plus précisément, les glycols désignent, dans le cadre de l'invention, les alkylèneglycols et les polyalkylèneglycols.

On entend par alkylèneglycol les alcools dihydroxylés dérivés d'hydrocarbures aliphatiques par remplacement de deux atomes d'hydrogène avec deux groupes hydroxyle. On préfère les (C₂-C₆) alkylèneglycol tels que l'éthylèneglycol et le propylèneglycol.
On entend par polyalkylèneglycol, les composés de formule

HO-(CₚH₂ₚO)ₙ-H

où p et n sont des entiers compris entre 2 et 6.

A titre d'exemple, on peut citer le dipropylèneglycol.

Selon l'invention, le groupe CₚH₂ₚO est linéaire ou ramifié. Le polyalkylèneglycol préféré selon l'invention est le dipropylèneglycol.

Les esters alkyliques d'acides carboxyliques préférés sont les esters d'alkyle en (C₁-C₆) d'acide (C₁-C₆)alcanoïque tel que l'acétate de butyle.

Lorsque la composition traitante comprend un sel acceptable sur le plan alimentaire, celui-ci peut être introduit dans la composition lors de sa préparation sous forme de sel ou sous forme neutre. Dans cette dernière hypothèse, le sel est formé *in situ* par addition d'une base appropriée, telle qu'un hydroxyde de métal alcalin (soude ou potasse).

La quantité de la solution selon l'invention devant être appliquée dépend essentiellement de la méthode d'application sélectionnée. Plus généralement, on ajuste la quantité appliquée de principe actif en fonction de la durée de stockage.

Les compositions traitantes sont préparées de façon conventionnelle en soi par simple mélange de leurs constituants, éventuellement sous agitation.

Les compositions selon l'invention doivent être appliquées une ou plusieurs fois. Selon un aspect avantageux, un traitement est effectué dans le silo, de préférence avant le remplissage ou sur les fruits secs, légumes secs, céréales, oléagineux aussitôt après récolte.

La quantité de composition traitante devant être appliquée dépend de leur nature des fruits secs, légumes secs, céréales, oléagineux concernés et du mode d'application sélectionné. Généralement, on applique entre 1 et 200 cm³ par m³ de stockage, préférentiellement entre 10 et 100 cm³/m³, étant entendu que ces doses sont rapportées à l'eugénol pur.

La composition peut être notamment appliquée par aspersion ou thermonébulisation. La solution est alors appliquée à température comprise entre 150° et 250°C, selon la nature de la composition. Ainsi, lorsqu'on applique de l'eugénol pur, la température est préférentiellement environ 230°C. Lorsque la composition A est appliquée, la thermonébulisation est préférablement réalisée à environ 180°C. Cette technique est connue en soi et est décrite dans FR 98 015305 et FR 99 04534.

La thermonébulisation est un procédé consistant à appliquer un brouillard extrêmement fin (dont les gouttelettes ont une taille de l'ordre du micromètre), lequel est produit par injection d'un liquide dans un courant d'air chaud, qui sert de véhicule à ladite composition traitante. Le brouillard ainsi produit permet une application homogène.

La thermonébulisation pourra avantageusement être mise en oeuvre par utilisation d'un appareil de thermonébulisation, tel que décrit dans FR 87 04 960 ou commercialisé sous la dénomination Electrofog Xeda^{®}. Cette machine de thermonébulisation électrique est constituée d'un ventilateur haute pression, d'une résistance électrique et d'une pompe volumétrique garantissant une régularité stricte des caractéristiques du brouillard produit et une introduction très progressive de la composition traitante dans la chambre de stockage.

De manière classique, les conditions permettant d'obtenir une taille de gouttes de 0,5 à 10 microns, notamment de l'ordre du micron, caractéristiques d'un brouillard de thermonébulisation, comprennent le chauffage de l'air à une température de 400° à 650°C avant l'injection du liquide.

Selon l'invention, la température du brouillard à la sortie de l'appareil de thermonébulisation est avantageusement choisie entre 110° et 300°C, de préférence entre 150° et 260°C, par exemple entre 170° et 250°C.

Cette variante est plus précisément décrite dans la demande FR 94 15 329.

Généralement, la thermonébulisation dans les silos est réalisée en plaçant le thermonébulisateur dans l'enceinte du silo, de préférence dans la moitié supérieure du silo, par exemple en plaçant le thermonébulisateur sur une plateforme placée au sommet de l'enceinte.

L'application peut être continue ou intermittente au cours de la durée du stockage.

De préférence, l'application est réalisée dans l'enceinte de stockage avant le remplissage ou répétée, tous les deux mois environ.

Les exemples suivants sont donnés à titre illustratif et non limitatif de la présente invention.

### EXEMPLE 1

### Protocole

### 1. Caractérisation préalable de la contamination naturelle de cellules vides de station de semences (Noaent-sur-Seine)

Six cellules (notées '20', 'L', '12', 'F', '8' et 'l') ont été étudiées. Ce sont des cellules en béton à fond conique (fond permettant de vider complètement la cellule et d'éliminer plus facilement les débris et poussières avant d'ensiler la récolte suivante). Les cellules identifiées par un chiffre ont une contenance de 350 t, celles identifiées par une lettre ont une contenance de 50 t. L'évaluation du niveau de contamination des six cellules a été réalisée par piégeage des spores ou des particules contaminées à l'aide d'un Airtest introduit dans la cellule par une trappe d'accès située à la base de la cellule.

Les boîtes de Petri de 90 mm de diamètre placées dans l'Airtest et constituant le piège contenaient un milieu Malt Agar + Triton ; l'ajout de triton modifie le développement des colonies fongiques qui restent compactes et sont plus faciles à dénombrer. Le volume d'air passant dans l'Airtest a été réglé sur 100 l, sauf pour une cellule dans laquelle nous avons testé 4 volumes d'air différents (10 l, 20 l, 40 l, 100 l). Pour chaque modalité, trois répétitions (3 boîtes de Petri) ont été réalisées.

Deux types de piégeage ont été effectués, un piégeage atmosphérique et un piégeage au niveau des parois des cellules de stockage. En effet, ces parois présentent des aspérités et peuvent retenir des spores de moisissures ou des particules infectées. L'Airtest a été placé à environ 5 mm de la paroi.

Pour évaluer le niveau de contamination des cellules de stockage des grains, une comparaison avec un témoin de contamination extérieure (4 répétitions) a été réalisée, par un piégeage atmosphérique à l'extérieur du silo (à environ 50 m des bâtiments du silo).

### 2. Etude sur deux cellules vides avant et après traitement avec l'eugénol

Sur deux cellules dont le niveau de contamination naturelle a été montré suffisant dans l'étude 1, des piégeages atmosphériques et des piégeages au niveau des parois ont été réalisés juste avant et 10 jours après traitement par thermonébulisation à l'eugénol. Cinq répétitions ont été effectuées (5 boîtes de Petri) pour chaque modalité.

Le traitement par thermonébulisation à l'eugénol a été effectué au niveau d'une plateforme sur laquelle était installé le thermonébulisateur. Après thermonébulisation, les cellules sont restées fermées pendant 10 jours pour garantir une bonne efficacité du traitement.

Compte tenu de la difficulté de manipulation des boîtes de Petri dans un environnement aussi contaminé qu'un silo, un témoin supplémentaire ("témoin de manipulation") a été réalisé. Quatre répétitions (4 boîtes de Petri) ont été faites pour ce témoin.

### Résultats

### 1. Caractérisation préalable de la contamination naturelle de six cellules vides

Les six cellules étudiées montrent un très fort niveau de contamination en *Penicillium* spp. On note une contamination en moisissures du genre *Aspergillus* dans la cellule 12. En moyenne, respectivement 77 et 85 *Aspergillus* spp. se développent sur les boîtes de Petri provenant de l'atmosphère et des parois de la cellule 12, alors qu'on n'en révèle le plus souvent aucun et au maximum 17 dans les autres cellules (tableau 1). Cette contamination, bien que nettement plus faible que la contamination par les *Penicillium* spp., n'est pas à négliger car les *Aspergillus* spp. sont également des producteurs potentiels d'ochratoxine A.

La comparaison de la contamination en *Penicillium* spp. des cellules vides avec celle du témoin extérieur est frappante (tableau 1). On ne révèle en moyenne que 1,25 colonie de *Penicillium* spp. et aucune colonie d'*Aspergillus* spp. Ce sont essentiellement des colonies de type *Cladosporium* spp. qui se développent à partir du piégeage atmosphérique extérieur.

Même si l'intensité de contamination en *Penicillium* spp. est très forte pour les six cellules, les cellules L et F apparaissent plus contaminées encore que les autres, en particulier au niveau de leurs parois (tableau 1). De manière générale, le piégeage au niveau des parois a donné plus de colonies de *Penicillium* spp. que le piégeage atmosphérique, sauf pour la cellule 8. Les parois des cellules de stockage constituent donc une source importante de contamination.

**Tableau 1 - Estimation du nombre moyen de colonies par boîtes de Petri obtenu après piégeage atmosphérique et piégeage au niveau des parois de 6 cellules de stockage vides**

| Cellule | Grain stocké | Volume Airtest | *Penicillium* Contamination atmosphérique | *Penicillium* Contamination des parois | *Aspergillus* Contamination atmosphérique | *Aspergillus* Contamination des parois |
|---|---|---|---|---|---|---|
| 20 | Blé | 100 l | 1560 | 3210 | 0 | 0 |
| L | Blé | 100 l | 2650 | 7500 | 0 | 0 |
| 12 | Blé | 100 l | 1240 | 1860 | 77 | 85 |
| F | Orge | 100 l | 2150 | 6480 | 3 | 0 |
| 8 | Orge | 100 l | 2020 | 1270 | 17 | 0 |
| I | Orge | 100 l | 3250 | 5900 | 0 | 0 |
| Témoin extérieur* | | 100 l | 1,25 | | 0 | |

| | | | | | | |
|---|---|---|---|---|---|---|
| * Piégeages atmosphériques effectués à l'extérieur des bâtiments du silo. | | | | | | |

D'autres champignons *(Cladosporium* spp.), ainsi que des bactéries et des levures contaminent également ces cellules, mais à un niveau moindre que les *Penicillium* spp. L'effet de volumes d'air croissants circulant dans l'Airtest sur le nombre de colonies piégées a été étudié dans la cellule I (Tableau 2).

**Tableau 2 - Estimation du nombre moyen de colonies par boîte de Petri obtenu après piégeages avec différents volumes de l'Airtest dans la cellule I**

| Volume Airtest | *Penicillium* Contamination atmosphérique | *Penicillium* Contamination des parois |
|---|---|---|
| 10 l | 2140 | 1190 |
| 20 l | 2680 | 2910 |
| 40 l | 2530 | 3640 |
| 100 l | 3250 | 5900 |

L'étude 1 a montré que le niveau de contamination en *Penicillium* spp. de ces six cellules est extrêmement fort et permet de tester aisément l'effet d'un traitement des cellules. C'est l'objet de l'étude 2 pour laquelle ont été retenues la cellule L, pour son niveau extrême de contamination, et la cellule 12 pour la présence d'un autre genre de moisissure, des *Aspergillus* spp.

### 2. Etude des cellules L et 12 avant et après traitement

Respectivement, 4 l et 20 l d'eugénol ont été thermonébulisés à 230°C (pendant ¾ h environ) dans la cellule L (≈50 m³) et dans la cellule 12 (≈ 350 m³).

L'évaluation de la contamination après traitement révèle une nette diminution du niveau de contamination en *Penicillium* spp. par le traitement dans les 2 cellules (tableau 3).

La contamination en *Aspergillus* spp. dans la cellule 12 est totalement maîtrisée par le traitement.

**Tableau 3 - Bilan de l'effet du traitement par thermonébulisation à l'eugénol sur la contamination atmosphérique et au niveau des parois de deux cellules vides**

| Cellule | Historique | Type piégeage (Airtest) | Volume Air(l) Airtest | Contamination en *Penicillium* (Nb colonies/boîte Petri) | | Contamination en *Aspergillus* (Nb colonies/boîte Petri) | |
|---|---|---|---|---|---|---|---|
| | | | | Avant traitement | Après traitement | Avant traitement | Après traitement |
| L | Blé | Atmosphère | 100 | 2650 | 106 | 0 | 0 |
| | | Parois | 100 | 7500 | 85 | 0 | 0 |
| | | | | | | | |
| 12 | Blé | Atmosphère | 100 | 1240 | 400 | 77 | 0 |
| | | Parois | 100 | 1860 | 207 | 85 | 0 |

II est à noter que la substance active peut s'accumuler dans le fond de la cellule. Ceci est tout à fait avantageux car la poussière et les menus débris, toujours présents dans les cellules de stockage et source importante de contamination, s'y accumulent également. Ils peuvent ainsi être traités efficacement.

### Conclusion

Les résultats ci-dessus démontrent que le procédé de thermonébulisation d'eugénol dans les cellules se montre très efficace contre les moisissures potentiellement productrices d'ochratoxine A, *Penicillium* spp. et *Aspergillus* spp., présentes dans les cellules de stockage de grain. L'efficacité porte à la fois sur la contamination atmosphérique et la contamination des parois des cellules.

## Revendications

1. Procédé pour décontaminer ou empêcher la contamination, par des mycotoxines et/ou les champignons ou moisissures les produisant, de silos de céréales, fruits secs, légumes secs, oléagineux, comprenant l'application auxdits réservoirs d'une composition comprenant de l'eugénol, de l'isoeugénol ou un de leurs sels acceptables sur le plan alimentaire et/ou de l'huile de girofle, ou leurs mélanges, par thermonébulisation, **dans lequel ladite composition est appliquée au silo vide.**

2. Procédé selon la revendication 1, tel que l'eugénol, l'isoeugénol, l'huile de girofle ou un de leurs sels acceptables sur le plan alimentaire, ou leurs mélanges, est appliqué à une dose comprise entre 1 et 200 cm³ d'eugénol par m³ de stockage.

3. Procédé selon la revendication 1 ou 2 pour lequel lesdites mycotoxines sont choisies parmi l'ochratoxine A, le désoxynivalénol, les aflatoxines, la zéaralénone, les trichothécènes, la fumosinine, la citrinine, l'acide pénicillique, la vomitoxine, la pateline.

4. Procédé selon la revendication 1, 2 ou 3 pour lequel lesdites mycotoxines sont choisies parmi l'ochratoxine A.

5. Procédé selon l'une quelconque des revendications précédentes, dans lequel ladite composition comprend, en pourcentage en poids :
- de 15% à 100 % dudit principe actif;
- de 0% à 10 % d'un ou plusieurs agents réduisant l'évaporation du principe actif ;
- de 0% à 85 % d'un tensioactif choisi parmi les tensioactifs anioniques, les tensioactifs non ioniques et leurs mélanges ; et
- de 0% à 80 % d'un solvant choisi parmi l'eau, les (C₁-C₆)alcanols, les (C₂-C₆)alkylèneglycol, les poly(C₁-C₆)alkylèneglycol, les esters (C₁-C₆) alkyliques d'acides (C₁-C₆)alcanoïque et leurs mélanges.

6. Procédé selon l'une quelconque des revendications précédentes dans lequel ladite composition est de l'eugénol pur ou de l'huile de girofle pure.

7. Procédé selon l'une quelconque des revendications précédentes, dans lequel ledit réservoir est contaminé ou présente un risque de contamination par *Penicillium ou Aspergillus.*

8. Procédé selon l'une quelconque des revendications précédentes, dans lequel la céréale est choisie parmi le blé, le maïs, le riz, l'orge.

9. Procédé selon la revendication **8,** dans lequel la céréale est le blé.

10. Procédé selon l'une quelconque des revendications 1 à **7,** dans lequel l'oléagineux est choisi parmi le tournesol, le colza, l'arachide.

11. Procédé selon l'une quelconque des revendications précédentes pour lequel lesdits céréales ou oléagineux sont sous forme de grains, plants, semences ou graines.

12. Utilisation d'une composition contenant de l'eugénol, de l'isoeugénol, un de leurs sels acceptables sur le plan alimentaire et/ou de l'huile de girofle ou leurs mélanges pour décontaminer ou empêcher la contamination, par des mycotoxines et/ou les champignons ou moisissures les produisant, de **silos** de céréales, oléagineux, légumes secs ou fruits secs, **dans lequel ladite composition est appliquée au silo vide,** par thermonébulisation.

## Claims

1. Method for decontaminating or preventing contamination, by mycotoxins and/or the fungi and/or moulds that produce them, of silos for cereals, dried fruits, dried vegetables, oil crops, which method comprises applying to said containers a composition comprising eugenol, isoeugenol or a nutritionally acceptable salt thereof and/or clove oil, or mixtures thereof, by thermal fogging, in which said composition is applied to the empty silo.

2. Method according to claim 1, such that the eugenol, isoeugenol, clove oil or a nutritionally acceptable salt thereof, or mixtures thereof, is applied at a dose of from 1 to 200 cm³ of eugenol per m³ of storage.

3. Method according to claim 1 or 2, in which said mycotoxins are selected from ochratoxin A, deoxynivalenol, aflatoxins, zearalenone, trichothecenes, fumosinin, citrinin, penicillic acid, vomitoxin, patulin.

4. Method according to claim 1, 2 or 3, in which said mycotoxins are selected from ochratoxin A.

5. Method according to any one of the preceding claims, in which said composition comprises, in wt.%:
- from 15% to 100% of said active ingredient;
- from 0% to 10% of one or more agents that reduce evaporation of the active ingredient;
- from 0% to 85% of a surfactant selected from anionic surfactants, non-ionic surfactants and mixtures thereof; and
- from 0% to 80% of a solvent selected from water, (C₁-C₆) alkanols, (C₂-C₆) alkylene glycols, poly-(C₁-C₆) alkylene glycols, (C₁-C₆) alkyl esters of (C₁-C₆) alkanoic acids and mixtures thereof.

6. Method according to any one of the preceding claims, in which said composition is pure eugenol or pure clove oil.

7. Method according to any one of the preceding claims, in which said container is contaminated or is at risk of contamination by *Penicillium* or *Aspergillus.*

8. Method according to any one of the preceding claims, in which the cereal is selected from wheat, corn, rice, barley.

9. Method according to claim 8, in which the cereal is wheat.

10. Method according to any one of claims 1 to 7, in which the oil crop is selected from sunflower, rape, groundnut.

11. Method according to any one of the preceding claims, in which said cereals or oil crops are in the form of grains, plants or seed.

12. Use of a composition comprising eugenol, isoeugenol, a nutritionally acceptable salt thereof and/or clove oil, or mixtures thereof, for decontaminating or preventing contamination, by mycotoxins and/or the fungi or moulds that produce them, of silos of cereals, oil crops, dried vegetables or dried fruit, in which said composition is applied to the empty silo by thermal fogging.

## Patentansprüche

1. Verfahren zur Dekontamination oder zur Verhinderung der Kontamination von Silos für Getreide, Trockenfrüchte, Trockengemüse, Ölfrüchte durch Mycotoxine und/oder die sie erzeugenden Pilze oder Schimmel, das die Anwendung einer Zusammensetzung auf diese Behälter umfasst, die Eugenol, Isoeugenol oder eines ihrer auf Nahrungsmittelebene akzeptablen Salze und/oder Nelkenöl oder ihre Mischungen umfasst und bei dem die genannte Zusammensetzung auf das leere Silo angewendet wird, durch Thermovernebelung.

2. Verfahren nach Anspruch 1, bei dem Eugenol, Isoeugenol oder eines ihrer auf Nahrungsmittelebene akzeptablen Salze oder ihre Mischungen mit einer Dosis angewendet wird, die zwischen 1 und 200 cm³ Eugenol pro Lagerungs-m³ enthalten ist.

3. Verfahren nach Anspruch 1 oder 2, bei dem die genannten Mycotoxine ausgewählt werden unter Ochratoxin A, Desoxynivalenol, den Aflatoxinen, Zearalenon, den Trichothecenen, Fumosinin, Citrinin, Penicillinsäure, Vomitoxin, Patulin.

4. Verfahren nach Anspruch 1, 2 oder 3, bei dem die genannten Mycotoxine unter Ochratoxin A gewählt werden.

5. Verfahren nach einem der vorangehenden Ansprüche, bei dem die genannte Zusammensetzung in Gewichtsprozent umfasst:
- von 15% bis 100% des genannten aktiven Prinzips;
- von 0% bis 10% eines oder mehrerer die Verdampfung des aktiven Prinzips reduzierenden Mittel;
- von 0% bis 85% eines Tensids, ausgewählt unter den anionischen Tensiden, den nichtionischen Tensiden und ihren Mischungen; und
- von 0% bis 80% eines Lösungsmittels, ausgewählt unter Wasser, den (C₁-C₆)alcanolen, den (C₂-C₆)alkylenglycolen, Poly(C₁-C₆)alkylenglycolen, den (C₁-C₆)alkylestern von (C₁-C₆)alkansäuren und ihren Mischungen.

6. Verfahren nach einem der vorangehenden Ansprüche, bei dem die genannte Zusammensetzung reines Eugenol oder reines Nelkenöl ist.

7. Verfahren nach einem der vorangehenden Ansprüche, bei dem der genannte Behälter eine Kontamination oder eine Kontaminationsgefahr durch Penicillin oder Aspergillus aufweist.

8. Verfahren nach einem der vorangehenden Ansprüche, bei dem das Getreide ausgewählt wird unter Weizen, Mais, Reis, Gerste.

9. Verfahren nach Anspruch 8, bei dem das Getreide Weizen ist.

10. Verfahren nach einem der Ansprüche 1 bis 7, bei dem die Ölfrüchte ausgewählt werden unter Sonnenblume, Raps, Erdnuss.

11. Verfahren nach einem der vorangehenden Ansprüche, bei dem die genannten Getreide oder Ölfrüchte Körnern, Pflanzen, Saaten, oder Samen sind.

12. Anwendung einer Eugenol, Isoeugenol oder eines ihrer auf Nahrungsmittelebene akzeptablen Salze und/oder Nelkenöl oder ihre Mischungen umfassenden Zusammensetzung zur Dekontamination oder zur Verhinderung der Kontamination von Silos für Getreide, Ölfrüchte, Trockenfrüchte, Trockengemüse durch Mycotoxine und/oder die sie erzeugenden Pilze oder Schimmel, bei der die genannte Zusammensetzung auf das leere Silo angewendet wird, durch Thermovernebelung.
